# EUROPEAN PATENT SPECIFICATION

(11) **EP 4 377 684 B1**
(45) Date of publication and mention of the grant of the patent: **21.10.2026**
(21) Application number: 22850313.2
(22) Date of filing: 28.07.2022
(51) Int. Cl.: G01N 27/26, G01N 27/27, G01N 27/00, C12Q 1/00, C12Q 1/26

(54) **ELECTROCHEMICAL BIOSENSOR FOR NITRITE OR NITRATE**
ELEKTROCHEMISCHER BIOSENSOR FÜR NITRIT ODER NITRAT
BIOCAPTEUR ÉLECTROCHIMIQUE POUR NITRITE OU NITRATE

(30) Priority: 28.07.2021 US 202163226411 P
(43) Date of publication of application: 05.06.2024
(73) Proprietor: Machado de Almeida, Maria Gabriela, 2605-658 Belas (PT)
(72) Inventor: MACHADO DE ALMEIDA, Maria Gabriela, 2605-658 Belas (PT); MONTEIRO, Tiago Carvalho, Cambridge CB1 2PU (GB)
(74) Representative: Couto, Cláudia
(86) International application number: PCT/US2022/038669
(87) International publication number: WO 2023/009720

(56) References cited:
- WO-A1-2012/176202
- WO-A1-2017/029034
- WO-A1-2019/224628
- WO-A1-2020/264347
- US-A- 5 356 786
- US-A- 5 443 701
- US-A1- 2005 186 333
- US-A1- 2005 246 849
- US-A1- 2009 061 524
- US-A1- 2010 304 499
- US-A1- 2011 212 533
- US-A1- 2014 322 715
- US-A1- 2016 202 272
- US-A1- 2016 356 738
- US-A1- 2019 064 165
- MONTEIRO TIAGO: "Development of point-of-care tests using enzyme (NiR & PON) based electrochemical biosensors", 1 April 2020 (2020-04-01), XP093210886, Retrieved from the Internet <URL:https://run.unl.pt/bitstream/10362/113413/1/Monteiro_2020.pdf>
- MONTEIRO TIAGO ET AL: "Bilirubin oxidase as a single enzymatic oxygen scavenger for the development of reductase-based biosensors in the open air and its application on a nitrite biosensor", BIOSENSORS AND BIOELECTRONICS, ELSEVIER SCIENCE LTD, UK, AMSTERDAM , NL, vol. 217, 15 September 2022 (2022-09-15), XP087188696, ISSN: 0956-5663, [retrieved on 20220915], DOI: 10.1016/J.BIOS.2022.114720
- MONTEIRO TIAGO, RODRIGUES PATRÍCIA R., GONÇALVES ANA LUISA, MOURA JOSÉ J.G., JUBETE ELENA, AÑORGA LARRAITZ, PIKNOVA BARBORA, SCHEC: "Construction of effective disposable biosensors for point of care testing of nitrite", TALANTA, ELSEVIER, AMSTERDAM, NL, vol. 142, 1 September 2015 (2015-09-01), NL , pages 246 - 251, XP093031025, ISSN: 0039-9140, DOI: 10.1016/j.talanta.2015.04.057

## Description

### FIELD OF INVENTION

The subject matter herein relates to an electrochemical biosensor and its uses.

### BACKGROUND

The 2020 doctoral thesis of Tiago Carvalho Monteiro (titled Development of point-of-care tests using enzyme (NiR & PON) based electrochemical biosensors) discloses point-of-care tests using electrochemical biosensors based on enzymes, namely, NiR and PON.

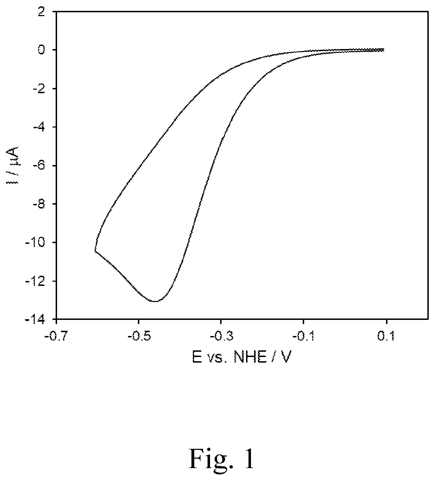

The commercialization of electrochemical biosensors that operate at low potentials (between -0.2 and -0.8 V vs standard hydrogen electrode [SHE]) has been hindered by the need to operate in anoxic conditions. Because molecular oxygen (O₂) is a major source of interference within this range of potentials, biosensors cannot be used in ambient air conditions. Specifically, the electrochemical reduction of dissolved oxygen (DO) to hydrogen peroxide (H₂O₂) or water (H₂O) at the electrode surface (see Fig. 1, and equations 1 and 2) produces an intense cathodic current (Fig. 1) that can mask the underlying redox reaction of the assay occurring at negative potentials, as is the case with bioelectrocatalytic reactions involving reductase enzymes. Additionally, the electrochemical reduction of DO can generate reactive oxygen species that might disrupt the catalytic activity of the assay.

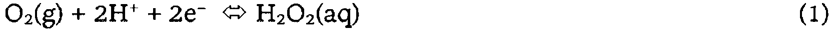

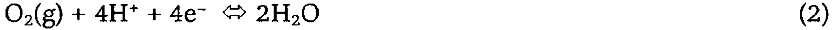

Molecular oxygen may also react directly with an enzyme involved in the biosensing reaction or with one of its reagents (e.g. natural substrate or co-substrate, redox mediators), thereby interfering with the bioanalytical process. Consequently, a test sample should be deoxygenated prior to performing an analytical measurement, especially for biosensors based on reductase enzymes.

Oxygen depletion technologies have been developed in other industries, for example, to protect packaged foodstuff from deterioration or to reduce pipeline corrosion. However, many of these developed solutions are likely unsuitable for biosensors. Rather, suitable oxygen depletion technologies for electrochemical biosensors are likely limited to physical methods, chemical methods, and biochemical methods.

Physical methods include solutions that are deoxygenated by vacuum degassing or bubbling inert gases (ca. 1 min/mL) to substitute dissolved gases from the internal atmosphere. Applicant observes that Argon/Nitrogen bubbling is the most commonly used method for oxygen purging in (bio)electrochemical applications. However, the technology is costly and not portable, meaning that it is not compatible with on-site monitoring or point-of-care testing. Furthermore, purging is the least effective method to degas solvents, and it is not possible to completely eliminate oxygen interference in ambient conditions. To completely eliminate oxygen interference, DO concentrations much be reduced from mg/L levels in ambient air (normal DO concentration is 0.3-3%) down to µg/L levels.

Chemical methods involve the addition of chemical compounds that directly react with and eliminate O₂, thus creating anoxic conditions. Applicant submits that chemical oxygen scavengers (e.g., ferrous iron, hydrazine, ascorbic acid, sodium sulfite, catechol, carbohydrazide, β-ketogluconate, gallic acid and photosensitive polymers) are likely more effective than physical methods since they can reduce the levels of DO below 0.01%. To the best of Applicant's knowledge, the only chemical reducing agent that has been tested in biosensing applications is sodium sulfite, which reacts with DO to form sodium sulfate. It has been reported that a nitrate reductase biosensor has been developed using sulfite as the oxygen scavenger for the measurement of nitrate in ambient air conditions. However, Applicant submits that sodium sulfite is not practical for commercial applications - anoxic conditions cannot be maintained for a sufficient period of time, as oxygen quickly diffuses back into solution. High concentrations of sulfite are required which can also interfere with normal enzyme activity.

Ascorbate is a well-known alternative chemical scavenger; however, oxidation of ascorbate to dehydroascorbic acid occurs slowly in the absence of a metallic catalyst. Other chemical scavengers may also interfere with the underlying electrochemical reaction of the test analyte or directly damage the biorecognition element of the biosensor. Many chemical scavengers are also toxic to the environment. Therefore, Applicant submits that direct chemical methods are not suitable for the development of commercial electrochemical biosensors.

Biochemical methods involve the addition of enzymatic oxygen scavengers to buffered assay solutions to eliminate DO. The typical biochemical approach is bienzymatic, with both an oxidase enzyme (and its chemical substrate - reducing agent) and catalase enzyme. With this approach, DO is first consumed as a co-substrate of the main oxidation reaction producing hydrogen peroxide; the hydrogen peroxide generated in this step then should be eliminated by catalase to yield water.

Applicant observes that glucose oxidase/glucose is the most common enzyme/substrate coupling that is used in first oxidation reaction of bienzymatic oxygen scavengers, but ethanol oxidase, galactose oxidase, pyranose 2-oxidase, and lactate oxidase (and their respective chemical substrates) have also been reported as viable alternatives. The resulting oxidation reaction releases reactive oxygen species, specifically H₂O₂, as a by-product. H₂O₂ should be quickly removed from the reaction solution by a second enzyme catalase. Catalase dismutates each mole of H₂O₂ back to one mole of water and one-half mole of oxygen. The O₂ molecules regenerated in the process are further reduced by the oxidase, so two glucose moles are consumed per oxygen mole in the net reaction. The product of glucose oxidation, D-glucono-1,5-lactone, is spontaneously converted to gluconic acid, which needs to be neutralized to avoid a drop in pH which can lead to enzyme deactivation. To this end, a high buffer concentration should be used within the limits of ionic strength.

The bienzymatic scavenger depletes DO from solution in ambient conditions in a reasonable time period for an electrochemical assay, and without affecting the biorecognition element of the biosensor. This approach has been successfully used by others in the development of a number of cathodic enzyme-based biosensors, including those based on DMSO reductase, nitrate reductase, and trimethylamine N-oxide reductase, and could be used with others such as perchlorate reductase, peroxidases, and cytochrome c. Applicant has also developed a nitrite assay (nitrite reductase) using a bienzymatic oxygen scavenger. Similar biochemical oxygen scavengers could also be used for reactions with a variety of electrode configurations, such as microelectrodes, microarrays, and electrodes, and lithography.

Although a bienzymatic oxygen scavenger for reductase-based biosensors is feasible, this approach is still not optimal for a commercial biosensor. For example, H₂O₂ is a very reactive molecule that can impact the electrode surface and other reagents in the assay if not efficiently eliminated. Catalase is not fully stable, and any loss of activity of the catalase enzyme would lead to the buildup of overwhelming levels of H₂O₂. H₂O₂ itself may also be electroactive in the negative potential window and interfere with the test results. In addition, commercially available glucose oxidase contains small amounts of free flavine, a redox cofactor. Flavine is electrochemically reduced at negative potentials, and can interfere with an electrochemical assay, especialy with low target analyte concentrations. In fact, Applicant observed the electrochemical signal of flavine (around -0.25 V) in a nitrite assay using a glucose oxidase-catalase bienzyme approach.

Accordingly, Applicant recognizes that a more effective and efficient biochemical oxygen scavenger system is needed to overcome the aforementioned shortcomings of existing approaches, which render them unsuitable for commercial biosensing applications. The present invention fulfills this need among others.

### SUMMARY OF INVENTION

The present invention relates to an electrochemical biosensor comprising a substrate which is overlaid with a working electrode which is overlaid with a nitrite or nitrate reductase,
wherein ascorbate oxidase (AOx) or bilirubin oxidase (BOD) overlays the nitrite or nitrate reductase, wherein a hydrophilic lid with a layer of ascorbate or bilirubin is placed above the layer(s) of AOx or BOD, respectively, to define a microfluidic channel there between.

The nitrate reductase in the biosensor is a periplasmatic (Nap), respiratory (Nar) or assimilatory (Nas) nitrate reductase.

The invention also relates to uses of the electrochemical biosensor for sensing nitrite or nitrate.

### BRIEF DESCRIPTION OF FIGURES

Fig. 1. Molecular oxygen (O₂) reduction at a pyrolytic graphite electrode. The cyclic voltammogram was measured in aerated phosphate buffer (50 mM, pH 7.0), at a scan rate of 50 mV s-1.
Fig. 2. Cyclic voltammograms of BOD (26.1 U mL-1) free in air-saturated solution (50 mM Tris-HCl, 25 mM PB, 50 mM KCl, pH 7.6), recorded in the (A) negative and (B) positive potential ranges at a scan rate of 20 mV s-1, using carbon SPE. (···) No enzyme in solution; (-) enzyme added.
Fig. 3. Cyclic voltammograms of electrolyte solutions (100 mM Tris-HCl buffer, pH 7.6, 100 mM KCl) containing the chemical substrates (A) ABTS, (B) ferricyanide and (C) ascorbate. Measurements were performed (···) without the enzyme and (-) 5 min. after its injecting BOD enzyme into the electrochemical cell. Cell volume, 2 mL. Scan rate, 20 mV s-1.
Fig. 4. Minimal concentration of ascorbate needed to promote oxygen depletion using the activity of BOD immobilized on SPE. The initial substrate concentrations ([S]i) ranged from 0 - 10 mM, in 100 mM Tris-HCl (pH 7.6), 100 mM KCl. Assays were duplicated.
Fig. 5. AOx/ascorbate oxygen scavenger system in a 50 µL electrolyte drop under ambient air: (A) cyclic voltammograms (20 mV s-1 scan rate) of 10 mM ascorbate (-) before and (· · ·) after the addition of the enzyme (125 U mL-1); (B) Monitoring of the oxygen purge reaction over 10 min., with the inset showing the amplified voltammograms from 0 to -0.8 V.
Fig. 6. Control assay with bare SPE and 10 mM ascorbate over 10 min in the absence of AOx. Cyclic voltammograms were plotted at a scan rate = 20 mV s-1. Electrolyte solution, 100 mM Tris-HCl buffer, pH 7.6, 100 mM KCl.
Fig. 7. Cyclic voltammograms recorded under ambient air with a 50 µL drop of electrolyte solution (100 mM Tris-HCl buffer, pH 7.6, 100 mM KCl) containing 10 mM ascorbate placed over an AOx/SPE. Voltammograms were recorded at the following timepoints: (a) 0, (b) 2, (c) 5 and (d) 10 min. after placing the drop over the SPE. Inset shows an amplification of the voltammograms between -0.1 and -0.8 V. Scan rate = 20 mV s-1.
Fig. 8. Schematic representation of the ccNiR/BOD-modified electrode. Detection of nitrite (NO2-) is accomplished according to the catalytic reaction mechanism (EC'), where ccNiR is first reduced by the electrode in the reversible electrochemical reaction (E), and afterwards it is oxidized in the irreversible chemical reaction (C') with nitrite.
Fig. 9. Electrochemical response of the BOD/ccNiR/SPE biosensors to increasing nitrite concentrations (0-200 µM) in a electrolyte solution (100 mM Tris-HCl buffer, pH 7.6, 100 mM KCl), containing 10 mM ascorbate (50 µL drop). Cyclic voltammograms were recorded at a 20 mV s-1 scan rate, under ambient air.
Fig. 10. Schematic representation of the localization of nitrate reductases in prokaryotic cells: NarGHI anchored to the membrane, NapAB in the periplasm, and Nas in the cytoplasm. The brown cubes represent the [4Fe-4S] centers and the blue cube the NarH [3Fe-4S] cluster. Nas is very diverse in terms of number and type of electron transfer centers for different organisms, and only NasA is represented.
Fig. 11. Direct electrochemistry monitored by cyclic voltammetry of nitrate reductase in the presence of sodium nitrate.
Fig. 12. Commercial dipstick analytical sensitivity. Percentage nitrite positive vs. nitrite concentration. Eight technologists tested each solution of urine. Chemstrip 8 results are shown by squares (■) and N-Multistix results are shown by triangles (▲).
Figs. 13A and 13B are perspective and cross-sectional views of a disposable nitrite biosensor of the present invention based on ascorbate oxidase/ascorbate oxygen scavenger, with ascorbate reagent in microfluidic channel.

### DETAILED DESCRIPTION

Herein disclosed, but not claimed, is a method of performing an electrochemical assay comprising reducing oxygen interference by introducing a biochemical oxygen scavenger to reduce dissolved oxygen directly into water. In one embodiment, the biochemical oxygen scavenger does not produce hydrogen peroxide or a reactive oxygen species when scavenging oxygen.

The biochemical oxygen scavenger is a single enzyme oxygen scavenger. The single enzyme oxygen scavenger is a multicopper oxidase (MCO) enzyme oxygen scavenger that comprises at least one of an ascorbate oxidase (AOx), or a bilirubin oxidase (BOD). Applicant has demonstrated the effectiveness of this approach using each of these MCO enzymes as a biochemical oxygen scavenger for the detection of nitrite with a reductase-based electrochemical biosensor. More specifically, AOx catalyzes the oxidation of ascorbate to dehydroascorbate via disproportionation of the semidehydroascorbate radical. The enzyme may be obtained from several sources, including, for example, plants, fungi, and eubacteria, and is commercially available. BOD catalyzes the oxidation of bilirubin to biliverdin, and can also oxidize other tetrapyrroles, phenols, and aryl diamines. AOx and BOD enzymes are advantageous because they have high enzyme activity at neutral pH, are stable, and have low sensitivity to chloride ions.

As electron donors, both AOx and BOD were coupled to the one of three chemical substrates (reducing agents): 2,2'-azino-bis(3-ethylbenzothiazoline-6-sulfonic acid) diammonium salt (ABTS), ferrocyanide, and ascorbate. The reactions are described below.

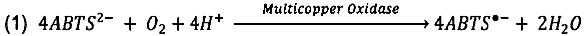

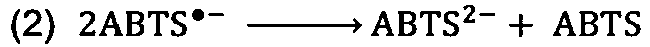

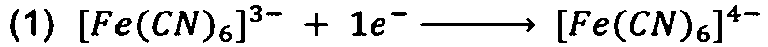

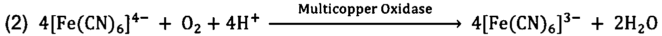

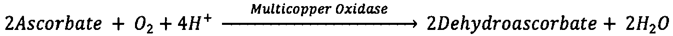

### Biomedical Applications

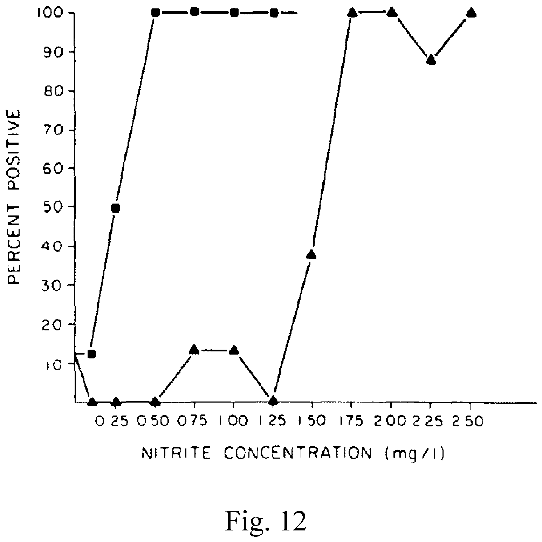

Urinary tract infection (UTI) is the most common cause of the presence of nitrite in urine, and the detection of urinary nitrites can be used as an indicator in the diagnosis of UTI. Normal urine does not contain any nitrite, which results from the breakdown of nitrate to nitrite by bacterial nitrate reductase enzymes. Nitrate reductase enzymes are present in most gram-negative and some gram-positive bacterial organisms, most commonly E. coli. The detection of nitrite in urine is highly specific for UTI but not a sensitive measure of infection, especially since not all organisms are nitrate-reducing. Therefore, the test is commonly used in conjunction with the leukocyte esterase test. Referring to Fig. 12, the sensitivities of commercial colorimetric 'dipstick' assays, for example, Chemstrip 8 (Biodynamics, Indianapolis, IN; now Roche Diagnostics) and N-Multistix (Ames Company, Elkhart, IN; now Siemens Diagnostics), were reported in comparison to a quantitative colorimetric research assay (limit of detection 0.1 mg/L nitrite). The analytical sensitivity of the Chemstrip 8 and N-Multistix were 0.25 mg/L and 1.5 mg/L, respectively. There was no difference in clinical sensitivity between Chemstrip 8 and the quantitative assay in 786 urine samples tested; however, the sensitivity of Chemstrip 8 and the quantitative assay were higher than that of N-Multistix. Therefore, analytical sensitivity of 0.25 mg/L or better is likely optimal for a clinical nitrite assay.

### Ecological Applications

Nitrate and nitrite biosensing has practical applications in the monitoring of the environmental and health risks posed by the levels of these substances in the food and water supply. Nitrate and nitrite salts have been used for a long time as food additives, especially in meats, fish, and cheeses, to protect from food-borne illness and improve taste. Nitrogen-containing fertilizers in fields and anthropogenic conversion of atmospheric nitrogen from combustion processes can also lead to substantial contamination of surface waters and groundwater supplies. Nitrate and nitrite are also present naturally in plants, soils, and waters. Concerns regarding human exposure to nitrate and nitrite through regular daily intake of nitrogen-containing foodstuffs began 40-50 years ago. There is a concern regarding the potential role of nitrite in forming genotoxic compounds, more specifically carcinogenic N-nitroso compounds via a reaction with secondary amines. While nitrate may be relatively safe, it is easily reduced to nitrite by bacteria in soil or within the digestive system. Excessive intake of nitrate and nitrites can also theoretically lead to irreversible oxidation of hemoglobin to methemoglobin - methemoglobin is unable to bind oxygen and, therefore, causes clinical cyanosis. Infants are particularly susceptible to nitrite-induced methemoglobinemia, a condition referred to as blue-baby syndrome (a small number of fatal cases have been linked to consumption of contaminated water resources). Although the link between nitrate/nitrite intake and health risks is not fully established, the concern is sufficient for the World Health Organization and European regulators to impose strict limits on the admissible levels of nitrate (50 ppm) and nitrite (0.1 - 3 ppm) in food products and drinking water. Nitrite levels may also pose risk to fish and other non-human wildlife. However, nitrate and nitrite levels in water and food supplies continue to be monitored using old-fashioned laboratory methods - biosensors may offer an improved real-world solution.

### Alternative Uses

In addition to nitrite reductase, the multicopper oxygen scavenger of the present invention can be utilized for the electrochemical detection of nitrate with an assay that employs immobilized nitrate reductase enzyme. Nitrate reductases are also key enzymes in the biological nitrogen cycle. Nitrate reductases perform the two-electron (two-proton) reduction of nitrate to nitrite in, with the release of one water molecule, according to the following reaction: 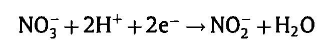 ${E}^{0} = + 420 mV vs . SHE$

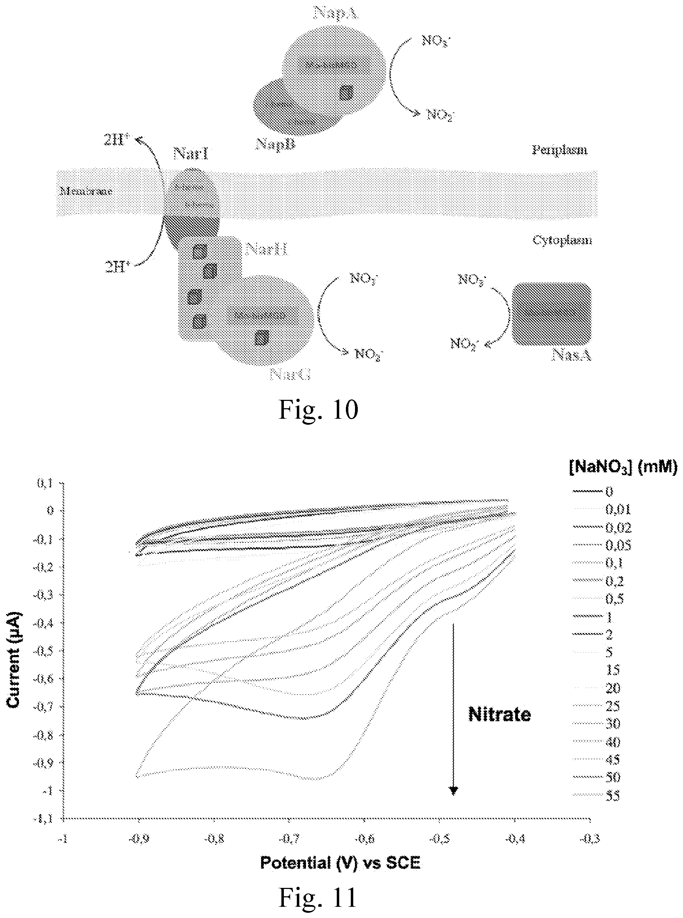

Prokaryotic nitrate reductases constitute a broad group of enzymes, belonging to the dimethyl sulfoxide reductase family of molybdenum-containing enzymes. They can be classified as periplasmic (Nap), respiratory (Nar), and assimilatory (Nas) nitrate reductases (see Fig. 10), according to their localization in cells (e.g. periplasm, membrane or cytoplasm, function (e.g. nitrate scavenging, anaerobic respiration) and molecular properties of the active site.

Several nitrate reductase enzymes have been studied by direct electrochemistry, with the protein adsorbed onto a solid electrode (e.g. NarGH from *Paracoccus pantotrophus* (*Pp*) and *Marinobacter hydrocarbonoclasticus,* NarGHI from *E. coli*; NapAB from *Pp* and *Rhodobater sphaeroides*; NarB from *Synechococcus* sp.). All nitrate reductase enzymes share a similar behavior in the presence of nitrate, i.e., a cathodic current is developed to represent the electrocatalytic reduction of nitrate.

Nitrate reductase from the sulfate reducing bacterium *D. desulfuricans* ATCC 27774 is a monomeric periplasmic enzyme. Applicant was able to adsorb this enzyme onto a pyrolytic graphite electrode and observe the direct electrochemical response to nitrate (Fig. 11) between a potential window from about -0.2 to -0.7 V vs normal hydrogen electrode (NHE). The electrochemical detection of this reductase enzyme also occurs in the key potential window for oxygen interference, so the implementation of this assay in the point-of-care setting may require implementation of an oxygen scavenging system as described in the present invention.

### Biosensor

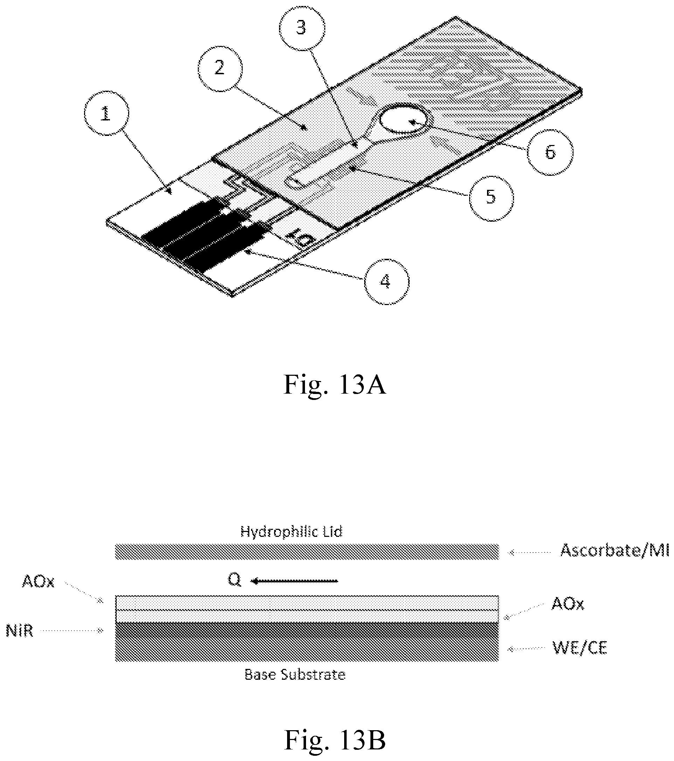

Referring to Fig. 13A and 13B, the biosensor of the present invention is shown. This biosensor has a base substrate 1, a lid 2, a microfluidic channel 3, connector pads 4, working electrode 5, and application zone 6. Referring to Fig. 13B, a schematic cross-section of the biosensor is shown. This particular cross section comprises a substrate, overlaid with a working electrode (WE/CE), which is overlaid with the nitrite reductase (NiR). The MCO enzyme oxygen scavenger is ascorbate oxidase (AOx), which overlays the nitrite reductase. A hydrophilic lid with a layer of ascorbate is placed above the layers(s) of AOx to define a microfluidic channel there between.

### Explanatory Embodiments.

Herein described is an electrochemical biosensor capable of reducing oxygen interference by introducing a biochemical oxygen scavenger to reduce dissolved oxygen directly into water. The oxygen is sufficiently reduced to avoid interference when operating at -0.2 and -0.8 V. The biochemical oxygen scavenger does not produce hydrogen peroxide or a reactive oxygen species when scavenging oxygen. The electrochemical assay is one of voltammetry, amperometry, or potentiometry. In one embodiment, the electrochemical assay is performed on a screen-printed electrode. Oxygen removal is achieved within 2 minutes and maintained for up to 5 minutes.

The single enzyme oxygen scavenger is a multicopper oxidase (MCO) enzyme oxygen scavenger, active at neutral pH and in the presence of chloride ions. The MCO enzyme is immobilized on the surface of a working electrode. The MCO enzyme is immobilized in a microfluidic channel. A sample is applied to a microfluidic channel contacts the MCO oxygen scavenger before reaching a working electrode. The MCO enzyme oxygen scavenger comprises at least one of an ascorbate oxidase (AOx), or a bilirubin oxidase (BOD). The ascorbate is immobilized around but not directly on the working electrode containing reductase enzyme. The ascorbate is immobilized in a microfluidic channel.

The electrochemical biosensor comprises a reductase enzyme. In one embodiment, the reductase enzyme is nitrite reductase. In one embodiment, the nitrite reductase is cytochrome c nitrite reductase (ccNiR). In another embodiment, the reductase enzyme is nitrate reductase.

In one embodiment, the single enzyme oxygen scavenger is an MCO enzyme co-immobilized as an outer layer on top of an inner layer of reductase enzyme on a working electrode. The electrochemical biosensor comprises a substrate, which functions as a reducing agent, coupled to the MCO enzyme oxygen scavenger, which is ascorbate, or bilirubin.

The invention relates to an electrochemical biosensor comprising a multicopper enzyme oxygen scavenger selected from ascorbate oxidase or bilirubin oxidase. In one embodiment, the biosensor further comprises a substrate comprising ascorbate.

The biosensor further comprises a reductase enzyme. In one embodiment, the reductase enzyme is nitrite reductase. In another embodiment, the reductase enzyme is nitrate reductase.

In one embodiment, the biosensor further comprises a screen-printed electrode. The ascorbate is immobilized in a dried state and is not exposed to oxygen or light. In one embodiment, an inner layer of the reductase enzyme is immobilized on a working electrode and an outer layer of the multi-copper enzyme is immobilized directly on top of the reductase enzyme.

Herein described, but not claimed, is a method of producing an electrochemical biosensor, comprising: depositing an inner layer of reductase enzyme on a working electrode and allowing it to dry; and subsequently, depositing a second layer containing a MCO enzyme on top of the reductase enzyme and allowing it to dry.

Herein described is the use of the electrochemical biosensor described above to detect the presence of nitrite in urine. The urine sample is applied to a microfluidic channel. The presence of a nitrite level of 0.05 - 1.50 mg/L (1 - 33 µM) or higher in urine indicates the presence of urinary tract infection. The electrochemical detection of urinary nitrite can be combined with the electrochemical detection of leukocyte esterase to identify the presence of bacterial infection in urine.

Herein described is the monitoring for the presence of nitrate or nitrite in water supply using the electrochemical biosensor described above to determine the level of nitrate or nitrite in said water supply. The water sample is applied to a microfluidic channel. The presence of nitrate above 50 ppm (800 µM) or the presence of nitrite above 0.1 - 3 ppm (2 - 65 µM) in a water sample indicates an unsafe level for human consumption.

Herein described is the monitoring the presence of nitrate or nitrite in soil or plant extracts, using the electrochemical biosensor described above to determine the level of nitrate or nitrite in said extracts.

Herein described is the monitoring for the presence of nitrate or nitrite in food extracts using the electrochemical biosensor described above to determine the level of nitrate or nitrite in said extracts.

### EXAMPLES

The following non-limiting examples are provided to show the efficacy of the present invention.

As a proof-of-concept, the single enzyme oxygen scavenger systems with AOx and BOD were implemented in a voltametric nitrite biosensor based on multihemic cytochrome c nitrite reductase (ccNiR), which performs the bioelectrocatalytic reduction of nitrite (NO₂⁻) to ammonia (NH₄⁺) when electrochemically activated at potentials below -0.3 V vs NHE. With optimization, the measurement of nitrite was successfully achieved with no interference from DO.

### Reagents and solutions

Analytical grade reagents, including ABTS, ascorbate, hydrochloric acid, potassium hexacyanoferrate (III), potassium phosphate, and sodium phosphate, potassium chloride, and potassium nitrite were obtained commercially. All solutions were prepared with deionized water (18 MΩ cm).

AOx from *Cucurbita* sp. (250 U) was purchased from Sigma-Aldrich. BOD from *Myrothecium verrucaria* (2.61 U mg⁻¹ solid) was provided by Amano Enzyme Inc. (Japan). ccNiR (300 U mg⁻¹) was purified from the sulfate reducing bacterium *Desulfovibrio desulfuricans* ATCC 27774. Stock solutions were prepared in 50 mM phosphate buffer, pH 7.6, in the following final concentrations:
- BOD, 52.2 U mL⁻¹
- AOx, 1.25 kU mL⁻¹
- ccNiR, 0.8 mg mL⁻¹

### Electrochemical measurements

Cyclic voltammetry was performed with a PGSTAT12 potentiostat from Eco Chemie Autolab using the control and data acquisition software GPES 4.9 (Eco Chemie). The electrode system consisted of disposable screen-printed electrodes (SPE) (DRP-C110) from Metrohm DropSens, composed by carbon working (ø = 4 mm) and counter electrodes, with a silver (Ag) pseudo reference electrode (328 mV vs SHE). For large volume assays (2 mL), an electrochemical cell was used, consisting of a glass cylinder containing the buffered supporting electrolyte and closed with a Teflon^{®} cap. A small strait cut was made in the center of the lid so that the SPE connections could pass outwards, while the electrodes were placed inside the cell.

The supporting electrolyte was 100 mM KCl in 100 mM Tris-HCl buffer (pH 7.6). Cyclic voltammograms were plotted at room temperature (22 ± 2 °C), from - 0.1 V to -0.8 V, at a 20 mVs⁻¹ scan rate, unless stated otherwise.

### Oxygen removal using BOD free in solution and different electron donors

First, the MCO substrates ABTS, ferrocyanide (after reduction of ferricyanide at the electrode), and ascorbate were tested individually as electron donors of BOD for the enzymatically catalyzed reduction of DO, having all components in solution. The chemical substrates (2.5 mM) were injected into the supporting electrolyte with a syringe, after which the enzyme BOD (5.22 U mL⁻¹) was added. The cyclic voltammograms were recorded immediately after the addition of each substrate into the electrochemical cell, and again 5 min after the addition of the enzyme.

### Oxygen depletion with Immobilized BOD and ascorbate in solution

SPEs were modified by dispensing 5 µL of BOD solution over the working electrode and allowing it to dry at room temperature for 40 min. The electrodes were stored dry at 4°C until use. The modified SPEs were covered with 50 µL of ascorbate solution (1, 2, 5 and 10 mM) prepared in the buffered supporting electrolyte, and cyclic voltammograms were recorded at the timepoints 0, 2 and 5 min., and then, every 5 min, up to 30 min. Each assay was duplicated.

### Oxygen depletion using AOx and ascorbate as reducing agent

The oxygen scavenging assay using AOx and ascorbate was tested in ambient air. The SPE was covered with a 45 µL drop of 10 mM ascorbate solution prepared in the buffered supporting electrolyte, and a CV was recorded from 0.8 to -0.8 V at a scan rate of 100 mV s⁻¹. Afterwards, 5 µL of AOx solution was gently mixed into the ascorbate drop with a micropipette, and CVs were recorded for 10 min, from 0.8 to - 0.8 V, at 20 mV s⁻¹. Alternatively, 5 µL of AOx solution was placed over the working electrode of the SPE and allowed to air-dry for approximately 1 h. A 50 µL ascorbate and electrolyte solution was then placed over the three-electrode system, and CVs were recorded in the same conditions as the assay performed with the enzyme free in solution. A control assay was run with fresh SPEs covered with 50 µL of 10 mM ascorbate solution without the presence of AOx enzyme

### Nitrite biosensors using BOD/ascorbate as oxygen scavenging system

To prepare the nitrite biosensors, the working electrodes were first coated with 5 µL of a ccNiR solution and air-dried for 40 min at room temperature. Subsequently, 5 µL of BOD solution were placed on the ccNiR-coated working electrode and air-dried for another 40 min. The resulting ccNiR-based biosensors were stored dry at 4°C until use.

The sensitivity of the biosensor for nitrite was determined by measuring the response to 50 µL of different standard solutions (0, 5, 50, 100, 150, and 200 µM) prepared in the buffered supporting electrolyte solution containing 10 mM ascorbate. Solutions were incubated for 2 mins before recording a CV, after which the electrode was discarded. The catalytic current (ΔI_{cat}) for each nitrite concentration was determined at - 0.5 V and -0.8 V, with the non-catalytic current (recorded in the absence of nitrite) being subtracted from all values. Each assay was performed in duplicate.

### Nitrite biosensors using AOx/ascorbate as oxygen scavenging system

To prepare the nitrite biosensors, three droplets (2 µL each) of ascorbate (2.45 M), were first placed around the working electrode and dried in an oven. The working electrodes were then coated with 5 µL of a ccNiR solution and air-dried for 40 min at room temperature (22 ± 2 °C). Subsequently, 5 µL of AOx solution (52.2 U mL⁻¹) was placed on the ccNiR-coated working electrode and air-dried for another 40 min. The resulting biosensors were stored dry at 4 °C until use.

The sensitivity of the biosensor for nitrite was determined by measuring the response to 50 µL of different standard solutions (one per SPE), with concentrations ranging from 0.5 to 200 µM. Cyclic voltammograms were recorded after an incubation time of 2 mins. Each assay was performed in duplicate. Cyclic voltammograms were plotted from 0 to -0.8 V at a 20 mV s⁻¹ scan rate. All current values were determined at the cathodic peak, and then plotted against the analyte concentrations.

### RESULTS

### Oxygen removal with BOD in solution by direct electron transfer (no chemical substrate)

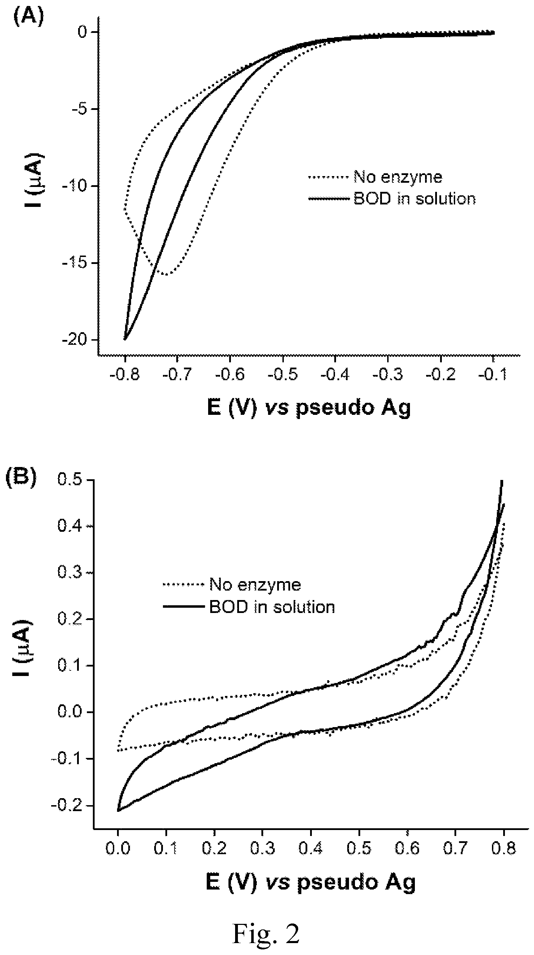

The BOD enzyme was added to solution in ambient air, and cyclic voltammograms were recorded in the negative and positive potentials windows using carbon SPEs. As observed in Fig. 2A, the cathodic peak of oxygen (*ca.* -0.7 V) shifted to more negative potentials in the presence of the enzyme (outside the working potential window), with an increase in current intensity (> 4 µA).

In the positive potential range (Fig. 2B), a distortion in the trace of the cyclic voltammogram could be observed when BOD was in solution, starting at 0.35 V in the cathodic scan, which was not present in the control assay (without enzyme). These effects on the CVs indicate that BOD was able to catalyze the reduction of oxygen directly while using the working electrode as an electron donor (direct electrochemical response). Such phenomena have been widely reported in carbon electrodes modified with MCO enzymes [Shleev *et al,* 2005; Li *et al,* 2014]; in this case, the current increase was greater than 0.1 µA, owing to the enzyme being immobilized and electrically wired on the transducing surface (direct electron transfer was greatly augmented in such conditions). However, the peak shift with direct electron transfer alone was not sufficient to eliminate interference from DO. To achieve full oxygen depletion, the effective reduction of BOD may require a chemical reducing agent (substrate) to serve as a redox partner, as described in the next section.

### Oxygen removal with BOD in solution using chemical electron donors

The oxygen scavengers were again tested in the same conditions, but now after adding one of the following chemical electron donors (substrates) to the electrolyte solution: ABTS, ferricyanide, or ascorbate. Taking into consideration the dissolved oxygen concentration in air-saturated solutions (*ca.* 0.2 mM), and a reaction stoichiometry of 1:4 oxygen/substrate (the O₂ reduction to water requires 4 electrons), an initial chemical substrate concentration of 2.5 mM was assumed to be sufficient for complete DO depletion.

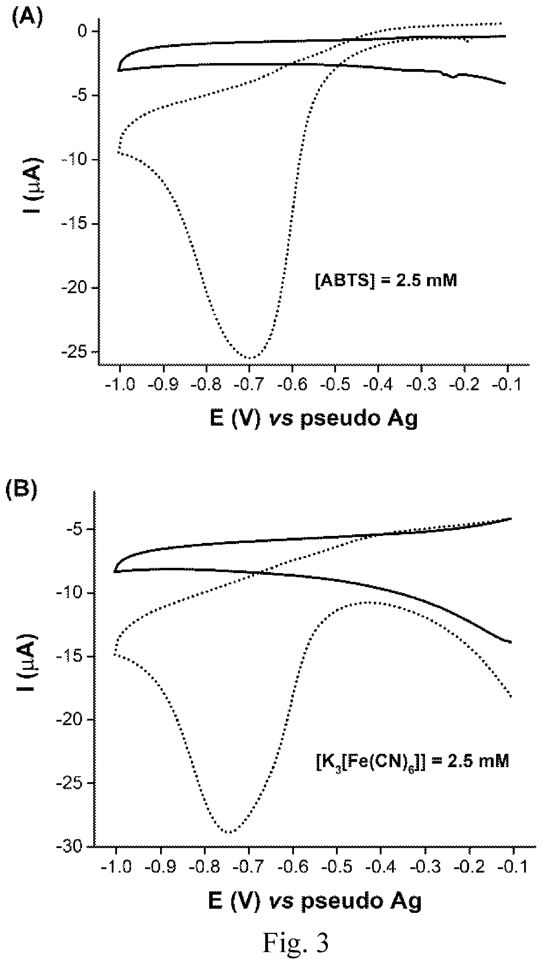

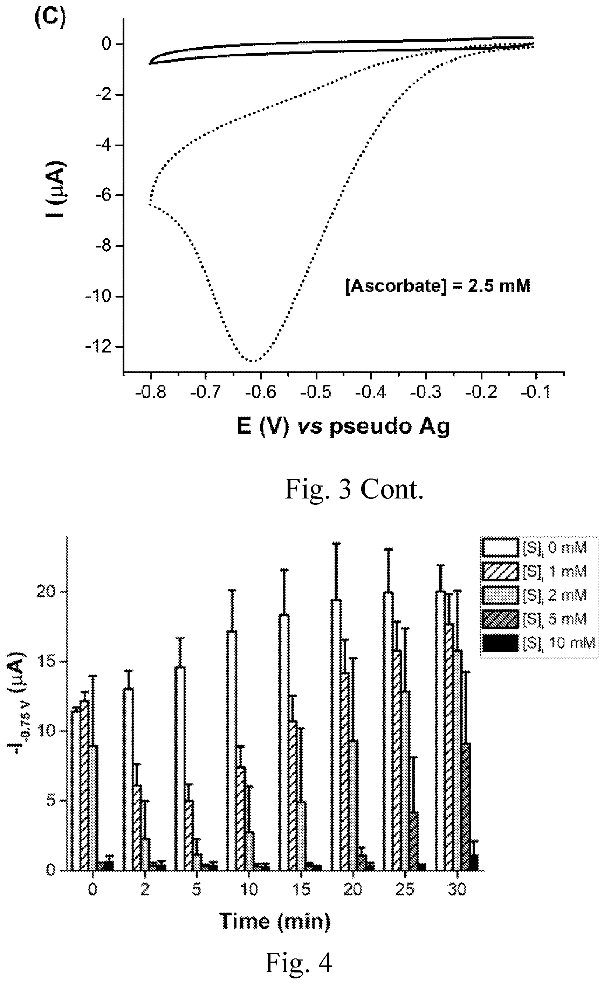

As shown in Fig. 3A and 3B, the addition of either ABTS or ferricyanide to aerated electrolyte solutions caused a slight increase in the cathodic peak of oxygen when compared to the control solution without substrate (Fig. 3A and B, dotted line). As for ferricyanide (Fig. 3C), the cathodic scan was distorted between -0.1 and -0.5 V as a result of the electrochemical reduction of FeCN₆³⁻ to FeCN₆⁴⁻ (E⁰' = 0.436 V *vs* NHE at pH 7). Even while the cathodic peak of ferricyanide occurs well outside the negative potential window, it is close enough for the diffusion-limited reduction to be a potential source of interference.

With all three chemical substrates, the addition of BOD enzyme to the solution made the broad cathodic wave assigned to the oxygen reduction disappear after 5 min. (Fig 4, dotted vs dashed trace). This is a clear evidence that the DO was fully consumed as a result of its enzymatic reduction to water in the presence of chemical reducing agents; thus, the potential electrochemical interference from oxygen was successfully eliminated

When comparing the CVs obtained with the three oxygen scavenging systems tested herein, BOD/ascorbate was the only one that produced a perfect background voltammogram. The FeCN₆⁴⁻/ FeCN₆³⁻ system caused a baseline distortion within the potential range -0.1 and -0.5 V. With the ABTS system, the capacitive current increased in the presence of the enzyme, probably due to the presence of ABTS²⁻/ABTS^{•}radicals resulting from the catalytic oxidation reaction [5-Tsujimura]. Furthermore, as the reaction went on to completion, the Ag reference electrode was chemically attacked and leached to the solution, since a metallic film could be observed leaving the surface of the ceramic substrate. This made the use of ABTS impractical as the chemical substrate for the single enzyme oxygen scavenger system. Accordingly, ascorbate was selected as the primary chemical substrate to promote the scavenging of oxygen with MCO enzyme, since it is not electroactive in the chosen potential range and provides a proper baseline in the full range of potentials.

### Oxygen removal with immobilized BOD and ascorbate in solution

Having demonstrated that the combination of BOD and ascorbate could effectively eliminate DO in an open electrochemical cell, the next step was to immobilize the enzyme on a SPE, and determine the optimal concentration of chemical substrate to promote anoxic working conditions in an open-air environment for a sufficient duration to allow for performance of a commercial reductase-based assay.

The BOD enzyme was drop cast on the working electrode, and the SPEs were covered with 50 µL of ascorbate solution at various concentrations (0, 1, 2, 5 and to 10 mM). The elimination of oxygen was monitored with cyclic voltammetry for 30 min by sampling the cathodic current at -0.75 V over time, and plotting current as a function of ascorbate concentration (Fig. 4). For the lowest concentrations (1 and 2 mM), the initial cathodic current went down to just 40% and 12% of the values obtained without enzyme, respectively. After a 5 min reaction time, the current increased progressively, meaning that the ascorbate had been fully consumed, with oxygen diffusing back into the solution. Therefore, these low substrate concentrations are not sufficient to purge DO from the solution. Quite the reverse occurred with 5 and 10 mM ascorbate, as the oxygen scavenger performed well with elimination of oxygen interference for at least 15 mins. A small increase in cathodic current was observed after 20 mins and 30 mins for 5 and 10 mM concentrations, respectively, due to ascorbate depletion. Clearly, the initial ascorbate concentration determines the operating interval under anaerobic conditions. Nevertheless, a time window of 15 min is more than sufficient to perform an analytical measurement.

### Oxygen scavenging using AOx and ascorbate in solution

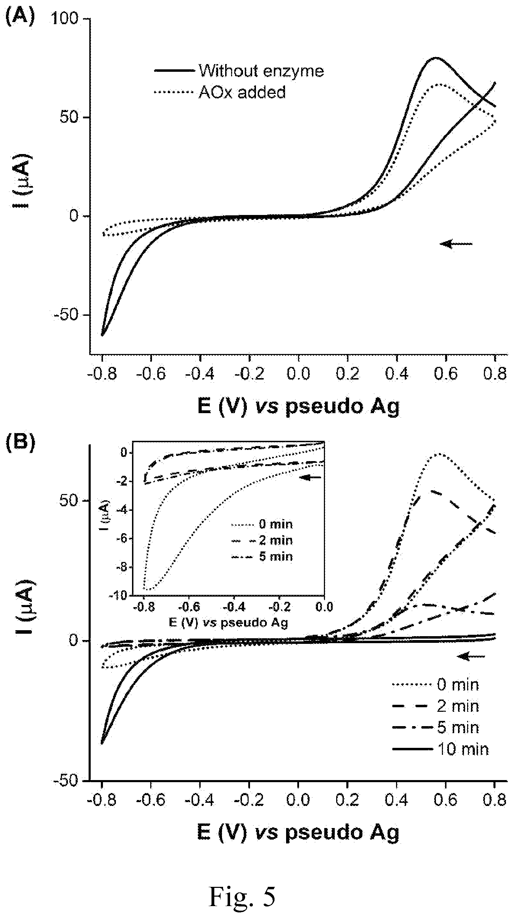

A second multicopper oxidase enzyme, AOx, was also tested as a single enzyme oxygen scavenger. Considering the previous results regarding the different types of electron donor substrates, ascorbate was the only chemical substrate tested. CVs were recorded within a broad potential range (-0.8; +0.8 V) in order to simultaneously observe the electrochemical signal of ascorbate and oxygen. In the absence of AOx, one anodic peak is observed at 0.55 V corresponding to the irreversible electrochemical oxidation of ascorbate to dehydroascorbate, and a cathodic wave between -0.4 and -0.8 V reflects the reduction of oxygen to water [Pisoschi *et al,* 2014; Tu *et al,* 2017]. With the addition of AOx enzyme into solution, the cathodic signal is quickly diminished with rapid elimination of oxygen, with complete elimination after just 2 mins Fig. 5 inset, dashed line). The anoxic conditions are maintained for at least 5 mins (Fig. 5 inset, dash-dotted line). The anodic peak for ascorbate also decreases slowly with depletion of the chemical substrate, and ascorbate is fully depleted after 10 mins with a reappearance of the cathodic oxygen interference (Fig. 5, full trace). This is explained by AOx catalyzing the oxidation of ascorbate coupled to the reduction of oxygen, consuming both reagents over time.

With eventual depletion of ascorbate, oxygen can diffuse back into the solution. Thus, a higher amount of ascorbate would be needed to purge oxygen from the solution for a longer period of time. Compared to the BOD/ascorbate system, the reaction with AOx/ascorbate oxygen scavenger occurs more quickly, which means that anaerobic conditions are achieved more quickly but also are maintained for shorter period of time. This is likely due to the higher number of enzyme units provided by AOx compared to BOD.

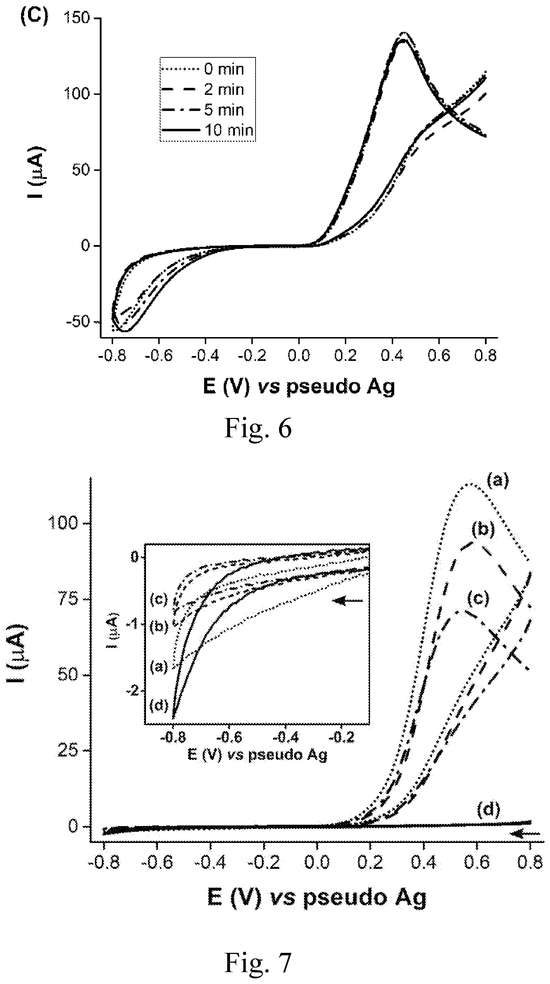

Control assay: Ascorbate itself is a known chemical oxygen scavenger. It has been shown that anaerobic conditions can be achieved at near physiologic pH in less than 1 min with 100 mM ascorbate, a concentration 10 times greater than the maximum concentration used in the single enzyme scavenger system. Therefore, a control assay was performed to analyze the direct deoxygenation capacity of 10 mM ascorbate under the same operating conditions as the single enzyme AOx system. As seen in Fig. 6, oxygen could not be scavenged from the solution, and the broad cathodic wave representing DO between -0.4 and -0.8 V remained unchanged over 10 mins. Also, the anodic peak representing ascorbate remained stable throughout the assay period and was not consumed without AOx present.

### Oxygen scavenging using immobilized AOx and ascorbate in solution

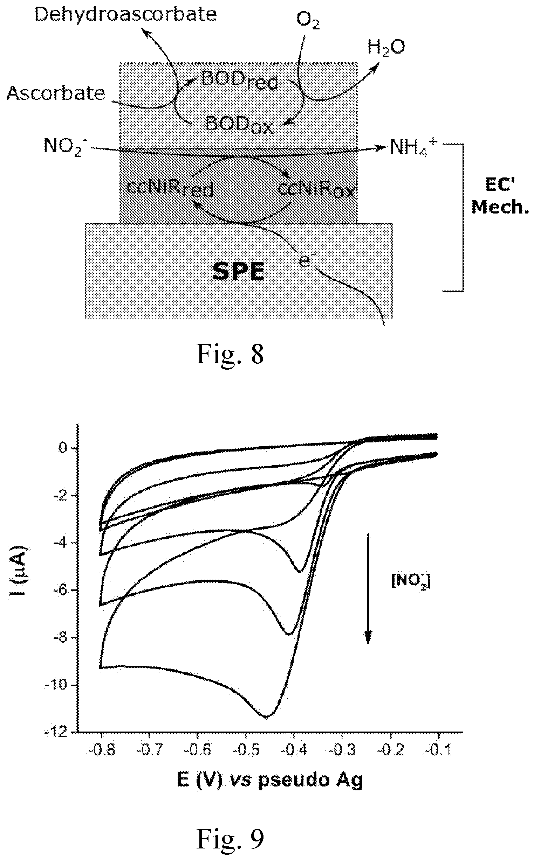

AOx was adsorbed onto the surface of the working electrode to assess oxygen scavenging in the presence of ascorbate solution. As shown in Fig. 7, enzyme immobilization did not compromise the capacity of the single enzyme scavenger to purge DO in ambient air. As with the free enzyme assay in solution, the immobilized enzyme assay demonstrated a rapid decrease in the cathodic current in the first 5 mins with a 10 mM ascorbate solution (inset of Fig. 7, a-c), again with a concomitant decrease in the anodic ascorbate signal (Fig. 7, a-c). After 10 mins, the ascorbate was completely depleted with complete absence of the anodic signal on the voltammogram (Figure 8, d) and, consequently, oxygen redissolution subsequently led to an in increase in the cathodic current near the inversion potential (-0.8 V). Despite the narrow operational window (2-5 min), this system is sufficient for performance of a reductase-based assay and can easily be extended by adjusting the concentration of ascorbate.

In conclusion, the combination of BOD or AOx enzyme with ascorbate provides a robust mono-enzymatic oxygen scavenger with maintenance of optimal conditions for reductase enzyme biosensors in ambient and at neutral pH.

### ccNiR-based nitrite biosensor

To demonstrate the feasibility of MCO enzymes in single-enzyme oxygen scavenger systems in reductase-based biosensors, BOD and AOx enzymes were co-immobilized with nitrite reductase (ccNiR) on SPEs by means of a simple sequential drop casting procedure.

### BOD/ascorbate as oxygen scavenging system

To construct nitrite biosensors, the working electrodes of carbon SPEs were modified with an inner layer of ccNiR and an outer layer of BOD. Then, the SPEs were covered with a small drop of solution containing nitrite and 10 mM ascorbate, as represented schematically in Fig. 8, enabling the detection of nitrite after just a 2 min incubation time.

As shown in Fig. 9, the catalytic current increased with nitrite concentration with no visible interference from DO in the cyclic voltammograms, a clear indication of the overwhelming effectiveness of the enzymatic oxygen scavenger in promoting anaerobic working conditions in ambient air during the time required for analyte detection.

### AOx/ascorbate as oxygen scavenging system

AOx was also co-immobilized on carbon-SPEs over a first layer of ccNiR. Prior to enzyme deposition, three droplets (2 µL) of a saturated ascorbate solution were placed around the working electrode and dried in an oven. The detection of the analyte was performed by placing a small drop of solution containing nitrite on the ccNiR/AOx-modified electrode, and, therefore, resuspending the dried ascorbate to drive the oxygen scavenging reaction. After a 2 min incubation period, cyclic voltammograms were recorded. With this setup, nitrite detection was also accomplished in ambient air with no oxygen interference and a limit of detection as low as 1 uM. This fully integrated system mimics a real-world application of the biosensor at the point-of-need without the requirement of adding ascorbate to the sample volume to maintain anoxic conditions. In addition to depositing the ascorbate around the working electrode, the ascorbate may be dried within a microfluidic channel to be resuspended in the fluid sample before reaching the working electrode.

## Claims

1. An electrochemical biosensor comprising a substrate which is overlaid with a working electrode which is overlaid with a nitrite or nitrate reductase,
wherein ascorbate oxidase (AOx) or bilirubin oxidase (BOD) overlays the nitrite or nitrate reductase, wherein a hydrophilic lid with a layer of ascorbate or bilirubin is placed above the layer(s) of AOx or BOD, respectively, to define a microfluidic channel there between.

2. The electrochemical biosensor according to any of the previous claims, wherein the nitrate reductase is a periplasmatic (Nap), respiratory (Nar) or assimilatory (Nas) nitrate reductase.

3. Use of the electrochemical biosensor of claim 1 for sensing nitrite or nitrate.

## Patentansprüche

1. Ein elektrochemischer Biosensor, umfassend ein Substrat, das mit einer Arbeitselektrode überzogen ist, die wiederum mit einer Nitrit- oder Nitratreduktase überzogen ist,
wobei Ascorbat-Oxidase (AOx) oder Bilirubin-Oxidase (BOD) die Nitrit- oder Nitratreduktase überlagert, wobei ein hydrophiler Deckel mit einer Schicht aus Ascorbat bzw. Bilirubin über der/den Schicht(en) aus AOx bzw. BOD angeordnet ist, um dazwischen einen mikrofluidischen Kanal zu bilden.

2. Der elektrochemische Biosensor gemäß einem der vorstehenden Ansprüche, wobei die Nitratreduktase eine periplasmatische (Nap), respiratorische (Nar) oder assimilatorische (Nas) Nitratreduktase ist.

3. Verwendung des elektrochemischen Biosensors nach Anspruch 1 zum Aufspüren von Nitrit oder Nitrat.

## Revendications

1. Le biocapteur électrochimique comprenant un substrat qui est recouvert d'une électrode de travail qui est recouverte d'un nitrite ou d'une nitrate réductase,
dans lequel une oxydase d'ascorbate (AOx) ou une oxydase de bilirubine (BOD) recouvre la nitrite ou la nitrate réductase, et dans lequel un couvercle hydrophile comportant une couche d'ascorbate ou de bilirubine est placé au-dessus de la ou des couches d'AOx ou de BOD, respectivement, de manière à définir un canal microfluidique entre ceux-ci.

2. Le biocapteur électrochimique selon l'une quelconque des revendications précédentes, dans lequel la nitrate réductase est une nitrate réductase périplasmique (Nap), respiratoire (Nar) ou assimilatoire (Nas).

3. Utilisation du biocapteur électrochimique selon la revendication 1 pour la détection du nitrite ou du nitrate.
